# EUROPÄISCHE PATENTSCHRIFT

(11) **EP 2 265 213 B1**
(45) Veröffentlichungstag und Bekanntmachung des Hinweises auf die Patenterteilung: **05.10.2011**
(21) Anmeldenummer: 09731538.6
(22) Anmeldetag: 30.01.2009
(51) Int. Cl.: A61F 2/00, A61F 2/02

(54) **IMPLANTIERBARER RESERVOIRKÖRPER**
IMPLANTABLE RESERVOIR BODY
CORPS DE RÉSERVOIR IMPLANTABLE

(30) Priorität: 15.04.2008 DE 102008018797
(43) Veröffentlichungstag der Anmeldung: 29.12.2010
(73) Patentinhaber: Dritte Patentportfolio Beteiligungsgesellschaft mbH & Co. KG, 12529 Schönefeld / Waltersdorf (DE)
(72) Erfinder: SCHRAG, Hans-Jürgen, 79877 Friedenweiler (DE)
(74) Vertreter: Zech, Stefan Markus
(86) Internationale Anmeldenummer: PCT/DE2009/000138
(87) Internationale Veröffentlichungsnummer: WO 2009/127176

(56) Entgegenhaltungen:
- EP-A- 1 886 628
- DE-A1- 10 013 519
- DE-A1-102004 018 807
- FR-A- 2 756 485
- US-A1- 2005 250 979

## Beschreibung

### Technisches Gebiet

Die Erfindung bezieht sich auf einen implantierbaren Reservoirkörper zur bedarfsgerechten Bereitstellung eines Arbeitsmediums, das zumindest zum Inflatieren einer implantierbaren Einheit dient.

### Stand der Technik

Implantierbare Reservoirkörper dienen der intrakorporalen Versorgung eines auf dem hydrodynamischen oder pneumatischem Wirkprinzip beruhenden implantierbaren Aktors mit einem Arbeitsmedium, das zur gezielten Aktorbetätigung über eine Fluid- bzw. Transmissionsleitung in ein aktorseitiges Füllvolumen überführt bzw. infiltriert wird oder in umgekehrter Weise aus dem aktorseitigen Füllvolumen den Reservoirkörper zurückgeführt wird. Der Austausch des Arbeitsmediums zwischen dem Reservoirkörper und dem aktorseitigen Füllvolumen erfolgt mit Hilfe einer miniaturisierten Pumpeneinheit, die über ein Steuerelektronikmodul bedarfsgerecht aktiviert wird.

Repräsentativ für eine Vielzahl bekannter Reservoirkörpersysteme seien nachfolgend einige konkrete Anwendungs- und Ausführungsbeispiele genannt.

Aus der DE 198 45 292 A1 ist ein Gerät zur Behandlung der Inkontinenz, d.h. der Unfähigkeit Harn oder Stuhlgang zu halten, beschrieben, das eine durch Mikropumpen gesteuerte Kompression der Harnröhre bzw. des Anus mittels eines in seinem Innendurchmesser variablen Implantates um das jeweilige Organ herum die natürliche Organaktivität zu unterstützen oder gar zu ersetzen vermag. Hierbei besteht das Gerät aus einem Reservoirkörper, der frei innerhalb des Körpers platzierbar ist, der über eine Fluid- bzw. Transmissionsleitung mit einem als Kompressionsring ausgebildeten Aktor verbunden ist, längs der eine Mikropumpeneinheit vorgesehen ist, deren Mikropumpenaktion und damit verbunden den Füllungsgrad des Kompressionsringes mittels einer von einem Anwender wählbaren Zeitdauer durch transkutane Energieversorgung der Mikropumpe bestimmt werden kann.

Ein weiteres Beispiel für die Anwendung eines implantierbaren Reservoirkörpers geht aus der DE 100 13 519 A1 hervor, in der eine implantierbare Sphinkterprothese beschrieben ist. Die Prothese weist zur steuerbaren Verengung der Harnröhre eine Manschette mit einem daran angebrachten Blähkörper auf, der im Wege einer Dillatation die Harnröhre abzuschließen vermag. Über eine Schlauchleitung ist der Blähkörper mit einem als flexibles Gefäß ausgebildeten Reservoirkörper verbunden, in dem als Arbeitsmedium eine Kochsalzlösung bevorratet ist. Längs der Schlauchleitung ist eine die Kochsalzlösung in geeigneter Weise fördernde Pumpeneinheit mit einer Steuer- und Versorgungseinrichtung vorgesehen, die subkutan unter die Haut appliziert wird, um auf kontaktfreie Weise einen induktiven Energie- und Signalaustausch zwischen einem extrakorporal positionierbaren Versorgungs- und Steuergerät und der implantierten Pumpeneinheit zu ermöglichen.

Ein weiteres Anwendungsbeispiel für einen implantierbaren Reservoirkörper ist der DE 10 2004 018 807 B4 zu entnehmen, in der ein implantierbares Schließmuskelprothesesystem beschrieben ist, das einen an der Innenseite eines elastischen Trägerringes angebrachten Kompressionscuff sowie einen an der Außenseite des Trägerringes angebrachten elastischen, sogenannten Reservoircuff vorsieht. Beide jeweils aus einem evakuierbaren, elastischen Material bestehenden Hohlkörper sind über eine Mikropumpe miteinander verbunden, die ein Transmissionsfluid je nach Kompressionsgrad des Kompressionscuffs zwischen beiden Hohlkörper hin und her fördert.

Alle derartige zu implantierenden Systeme unterliegen der Forderung nach einem möglichst kompakten und raumsparenden Design, das im Wege der Implantation beim Patienten möglichst keine oder nur geringfügige Irritationen hervorrufen sollte, obgleich es gilt die Funktionalität, Effektivität und letztlich auch die Lebensdauer derartiger Systeme zu optimieren. Besondere Bedeutung kommt in dieser Hinsicht dem zu implantierenden Reservoirkörper zu, aus dem es letztlich gilt, das für eine gewünschte Aktorik einer zu implantierenden Einheit erforderliche Arbeitsmedium in kontrollierter Weise zu Zwecken eines dosierten Inflatierens des aktorseitigen Füllvolumens bereit zu stellen. Ebenso gilt es aufgrund einer zumeist zyklischen Betriebsweise einer zu implantierenden Aktoreinheit einen kontrollierten Rückfluss des Arbeitsmediums in den Reservoirkörper zu Zwecken eines Deflatierens des aktorseitigen Füllvolumens zu gewährleisten.

### Darstellung der Erfindung

Der Erfindung liegt die Aufgabe zugrunde, einen implantierbaren Reservoirkörper zur bedarfsgerechten Bereitstellung eines Arbeitsmediums, das zumindest zum Inflatieren einer implantierbaren Einheit dient, zweckoptimiert im Hinblick auf Form, Größe und Funktionalität derart bereitzustellen, dass die durch die Implantation bei einem Patienten hervorgerufenen Irritationen auf ein Minimum reduziert werden. Des Weiteren soll es möglich sein, einen implantierbaren Reservoirkörper weitgehend unabhängig von der Funktionalität der mittels des Arbeitsmediums zu betreibenden implantierbaren Einheit auszubilden, so dass ein möglichst modularer Einsatz des Reservoirkörpers bzw. eine freie Kombinierbarkeit des Reservoirkörpers mit weitgehend unterschiedlich ausgebildeten implantierbaren Einheiten möglich sein soll.

Die Lösung der der Erfindung zugrunde liegenden Aufgabe ist im Anspruch 1 angegeben. Den Lösungsgedanken vorteilhaft weiterbildende Merkmale sind Gegenstand der Unteransprüche sowie der weiteren Beschreibung insbesondere unter Bezugnahme auf das illustrierte Ausführungsbeispiel zu entnehmen.

Dem lösungsgemäßen implantierbaren Reservoirkörper gemäß den Merkmalen des Oberbegriffes des Anspruches 1 liegt die Idee zugrunde, eine für die elektrische Energieversorgung sowie auch in vorteilhafter Weise für den Austausch elektrischer Signale erforderliche Induktionsspulenanordnung innerhalb des Reservoirkörpers zu integrieren. So ist ein elastischer Hohlkörper vorgesehen, dessen Hohlkörperwand das Reservoirvolumen umschließt, wobei die Induktionsspulenanordnung innerhalb des Reservoirvolumens platziert ist und/oder die Induktionsspulenanordnung oder eine weitere Induktionsspulenanordnung mit der Hohlkörperwand verbunden oder in der Hohlkörperwand integriert ist. Durch eine derartige Kombination des Reservoirkörpers mit wenigstens einer Induktionsspulenanordnung wird in vorteilhafter Weise die zumeist flächige Formgebung des Reservoirkörpers ausgenutzt, um die ebenfalls flächig ausgebildete Induktionsspulenanordnung in Form einer oder mehrerer flächig ausgebreiteter elektrischer Windungen platzsparend unterzubringen. In dieser Form lässt sich der Reservoirkörper subkutan unter die Hautoberfläche ins Fettgewebe implantieren, so dass der räumliche Abstand zwischen der Induktionsspulenanordnung und einer extrakorporal vorzusehenden Signal- und Energieversorgungseinheit möglichst klein ist, wodurch eine gute induktive Kopplung zur Energie- und Signalübertragung erzielt werden kann.

Einen besonders hohen Grad an Autarkie und technischer Funktionalität erhält ein lösungsgemäßer implantierbare Reservoirkörper, wenn gemeinsam mit der Induktionsspulenanordnung eine Pumpen-/Elektronikeinheit innerhalb des Reservoirkörpers vorgesehen ist, vermittels der das im Reservoirkörper bevorratete Arbeitsmedium dosiert und kontrolliert über einen Fluidkanal zu einer weiteren implantierbaren Einheit überführt werden kann. Die Pumpen-/Elektronikeinheit kann gleichsam der sich innerhalb des Reservoirkörpers befindlichen Induktionsspulenanordnung freischwimmend innerhalb des von dem elastischen Hohlkörper umschlossenen Reservoirvolumens eingebracht oder mit der Hohlkörperwand des Reservoirkörpers verbunden oder in dieser integriert sein. Zudem lässt sich auch die Batterieeinheit im Reservoirkörper geeignet unterbringen,

Alternativ bietet es sich an die Pumpen-/Elektronikeinheit außerhalb des Reservoirkörpers vorzusehen, beispielsweise längs eines aus dem Reservoirkörper führenden Fluidkanals, der den Reservoirkörper mit der zu implantierenden Einheit verbindet, um diese entsprechend mit dem im Reservoirkörper bevorrateten Arbeitsmedium zu inflatieren. In diesem Fall gilt es die Pumpen-/Elektronikeinheit mit bioverträglichem Material zu Verkapseln. Beispielsweise können in einer derartigen, getrennt von dem Reservoirkörper ausgebildeten Gehäusekapsel auch weitere Komponenten integriert werden, etwa ein Befüllungsport für das Befüllen des Reservoirkörpers mit dem Arbeitsmedium oder die Batteriereinheit zur Speicherung elektrischer Energie.

In einer bevorzugten Ausführungsform sieht der implantierbare Reservoirkörper im Bereich seiner Hohlkörperwand einen Wandabschnitt vor, der in Art einer Adapterplatte ausgebildet ist, die in vorteilhafter Weise aus einem gegenüber der elastischen Hohlkörperwand stabileren bzw. steiferen Material gefertigt ist. Die Adapterplatte dient einerseits zur Fixierung der ansonsten freischwimmend innerhalb des Reservoirkörpers befindlichen Induktionsspulenanordnung sowie auch für eine fixierte Durchführung eines Fluidkanals, der die Adapterplatte entweder vollständig durchragt oder der zumindest einseitig an dieser derart angebracht ist und in einer ventilartig ausgebildeten Verbindungsstruktur mündet, an der eine zu der implantierbaren Einheit weiterführende Transmissions- oder Fluidleitung adaptierbar ist. Die Adapterplatte kann auch als eine direkte Verbindungsschnittstelle zu weiteren implantierbare Komponenten verwendet werden. Hierzu sind an der Adapterplatte mechanische Verbindungsstrukturen für die Adaption wenigstens einer weiteren zu implantierbaren Einheit vorgesehen. Neben rein mechanischen Befestigungsstrukturen weist die Adapterplatte darüber hinaus auch wenigstens eine elektrische Verbindungsstruktur auf, um eine weitere zu implantierende Einheit elektrisch mit einer vorzugsweise innerhalb des Reservoirkörpers vorgesehenen Pumpen-/Elektronikeinheit samt Batterieeinheit zu verbinden.

Alternativ oder in Kombination zur Adapterplatte verfügte eine Ausführungsform des implantierbaren Reservoirkörpers über einen semizirkulären oder zirkulären Ringport zum Anstechen und Befüllen des Reservoirkörpers mit dem Arbeitsmedium, das vorzugsweise aus einer Kochsalzlösung besteht. Denkbar wäre auch die Verwendung eines gasförmigen Arbeitsmediums. Auch könnte parallel zum Fluidkanal ein Befüllungsport vorgesehen sein, an dem die Adaption einer Befüllungsleitung für den Reservoirkörper möglich ist. Üblicherweise werden Reservoirkörper im entleerten Zustand implantiert und nachträglich mit einem entsprechenden Arbeitsmedium über einen Befüllport befüllt.

In besonders vorteilhafter Weise besteht der Reservoirkörper aus einem vorgeformten Hohlkörper, vorzugsweise aus einem geeigneten Polyurethan, dessen Hohlkörperwand im Wege der In- sowie auch Deflation eine Materialstauchung oder Verformung bei weitgehend konstanter Hohlkörperwanddicke unterliegt, wobei über den vorstehend beschrieben Port das Arbeitsmedium in den evakuierten und implantierten Reservoirkörper injizierbar ist und die Pumpeneinheit zwischen evakuierten Reservoirkörper und der weiter zu implantierenden Einheit transferiert wird. Somit basiert die In- bzw. Deflation hauptsächlich auf einer Materialstauchung und Verformung des Hohlkörpers mit einem definierten Volumen bei weitgehend konstanter Wanddicke und nicht auf einer, wie üblicherweise bei den herkömmlichen, z.B. aus Silikon gefertigten Kompressionskörpern bestehenden Materialdehnung. Der Vorteil besteht neben der geringen Materialbeanspruchung in der Eliminierung des initialen Dehnungswiderstandes des Reservoirkörpers woraus sich ein Hysteresephänomen des Druckes während der Kompressionsfüllung ableitet. Diese Konstellation, in Kombination mit dem aus der integrierten Systemkonzeption resultierenden minimalen Totraumvolumen des Fluidkanals wirkt sich positiv auf die Leistungscharakteristik und das Energiemanagement der zu verwendenden Pumpeneinheit aus.

Der lösungsgemäß ausgebildete, implantierbare Reservoirkörper, der in Verbindung mit unterschiedlich ausgestalteten zu implantierenden Einheiten modular einsetzbar ist, wird ohne den allgemeinen Lösungsgedanken zu beschränken im folgenden anhand konkreter Ausführungsbeispiele beschrieben.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: schematisierte Darstellung eines lösungsgemäß ausgebildeten Reservoirkörpers mit integrierter Induktionsspulenanordnung sowie integrierter Pumpen-/Elektronikeinheit,
- Fig. 2a,b: Ausführungsbeispiel eines Reservoirkörpers mit über eine Adapter- platte verbundener Pumpen-/Elektronikeinheit.

### Wege zur Ausführung der Erfindung, gewerbliche Verwendbarkeit

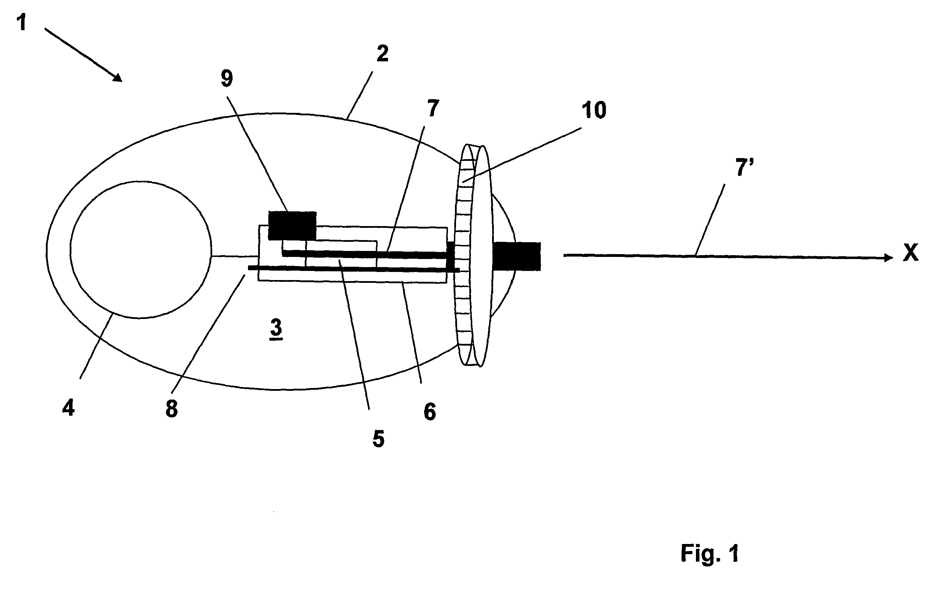

In Figur 1 ist stark schematisiert ein lösungsgemäß ausgebildeter Reservoirkörpers 1 dargestellt, der einen Hohlkörper 2 vorsieht, der ein Reservoirvolumen 3 umschließt. In dem Reservoirvolumen 3, die von der aus einem geeigneten Polyurethan gefertigten Hohlkörperwand des Hohlkörpers 2 umschlossen ist, sind weitgehend freilagernd bzw. freischwimmend eine Induktionsspulenanordnung 4, eine Pumpeneinheit 5, eine Elektronikeinheit 6, ein Fluidkanal 7 sowie ein Befüllungsport 8 vorgesehen. Zumindest die Induktionsspulenanordnung 4 kann alternativ zu der freischwimmenden Lagerung innerhalb des Reservoirvolumens 3 auch innerhalb der Hohlkörperwand des Hohlkörpers 2 integriert werden. Um zu vermeiden, dass die im Inneren des Reservoirkörpers 1 angebrachten Komponenten willkürliche räumliche Lagen einnehmen, wird vorgesehen, zumindest die Pumpeneinheit 5 sowie die Elektronikeinheit 6 auf einem gemeinsamen Trägersubstrat zu platzieren, das im Inneren des Hohlkörpers 2 mit der Hohlkörperwand an einer definierten Stelle verbunden bzw. fixiert ist. Zugleich bietet es sich an, den im Inneren des Reservoirkörpers 1 befindlichen Fluidkanal 7 als auch den Befüllungsport 8 als bauliche Einheit mit der Pumpen-/Elektronikeinheit 5, 6 zu verbinden. In besonders vorteilhafter Weise ist im offenen Ansaugbereich des Fluidkanals 7 ein Ansaugschutz 9 vorgesehen, der zum Schutz vor einem Verschließen mit einem innerhalb des Hohlkörpers 2 vorhandenen Oberflächenbereiches dient.

Zum Befüllen des Reservoirkörpers 1 ist der Portkanal 8 vorgesehen, der in herkömmlicher Bauart über eine plane Anstichfläche 10 oder eine zirkulär bzw. semizirkulär ausgeformte konvexe oder konkave Anstichfläche 10 mit einer externen Befüllvorrichtung verbunden werden kann. Die Anstichfläche 10 kann mit oder ohne ausgeformten Stichschutz für die Hohlkörperwand versehen werden.

In der in Figur 1 dargestellten Ausführungsform sei darüber hinaus angenommen, dass eine Batterieeinheit innerhalb der Elektronikeinheit 6 integriert ist, die zu Zwecken der Energiespeicherung im Wege eines elektrischen Ladevorgang, wie eingangs beschrieben, dient.

Der in Figur 1 dargestellte Reservoirkörper 1 stellt somit eine vollständig autark arbeitende Antriebseinheit für eine implantierbare Einheit X dar, die mit einem Arbeitsmedium, vorzugsweise von flüssiger oder gasförmiger Natur, betrieben wird und daher über eine Fluidleitung 7' mit dem Reservoirkörper 1 verbunden ist.

Typischerweise dient der lösungsgemäß ausgebildete Reservoirkörper 1 zur kontrollierten und dosierten Bereitstellung eines Arbeitsmediums für in- sowie auch deflatierbare implantierbare Einheiten X, mit denen ein kontrolliertes Verschließen von Hohlorganen, wie beispielsweise Blut-, Harn- oder Stuhl-fördernde Hohlorgane möglich ist. Ein bevorzugtes Beispiel einer derartig implantierbaren Einheit ist beispielsweise der eingangs zitierten DE 10 2004 018 807 B4 zu entnehmen.

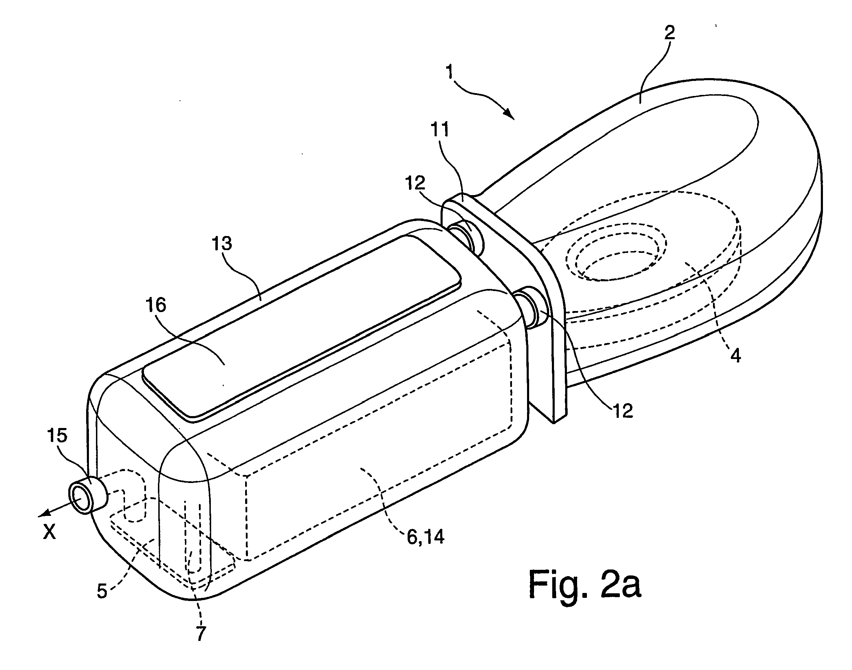

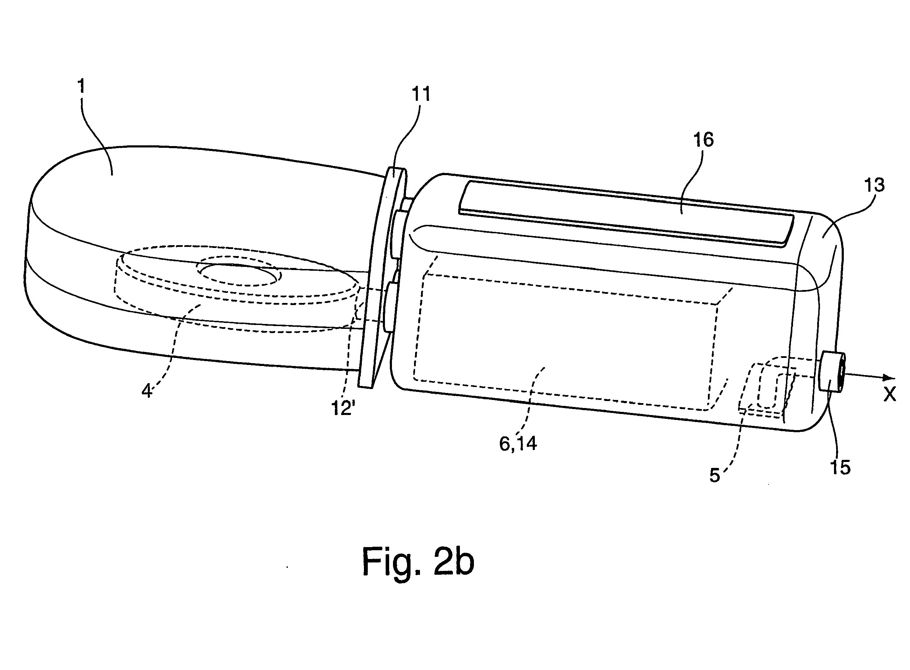

Im Unterschied zu dem in Figur 1 dargestellten Ausführungsbeispiel weist das in den Figuren 2a und b illustrierte Ausführungsbeispiel einen Reservoirkörper 1 auf, in dessen Reservoirvolumen lediglich eine Induktionsspulenanordnung 4 eingebracht ist. Der Hohlkörper 2 des Reservoirkörpers 1 schließt einseitig mit einer Adapterplatte 11 fluiddicht ab, an der diverse Anschlussstrukturen 12 vorgesehen sind, über die eine Batterieeinheit 14, die Elektronikeinheit 6 sowie die Pumpeneinheit 5, die innerhalb eines gekapselten Gehäuses 13 integriert sind, adaptierbar sind. Im Ausführungsbeispiel gemäß Figur 2b ist ersichtlich, dass die Induktionsspulenanordnung 4 über eine elektrische Verbindungsstruktur 12' mit einer innerhalb des Gehäuses 13 integrierten Batterieeinheit 14 / Elektronikeinheit 6 verbunden ist, die eine ebenfalls im Gehäuse 13 integrierte Mikropumpeneinheit 6 mit elektrischer Energie und entsprechenden Steuersignalen versorgt. Die Pumpeneinheit 5 ist innerhalb eines mit dem Reservoirkörper 1 verbundenen Fluidkanals 7 angebracht, der ausgangsseitig eine Anschlussstruktur 15 vorsieht, mit der eine mit dem innerhalb des Reservoirkörpers 1 bevorrateten Arbeitsmedium zu versorgende, implantierbare nicht weiter dargestellte Einheit X verbindbar ist. An der in Figur 2b dargestellten, oberen Seitenfläche des quaderförmig ausgebildeten Gehäuses 13 ist überdies ein Flächenport 16 vorgesehen, über den die Befüllung des Reservoirkörpers 1 erfolgen kann.

Das in den Figuren 2a und b illustrierte Ausführungsbeispiel zeigt eine einfache Ausbildungsform eines lösungsgemäß ausgebildeten Reservoirkörpers 1, in dem lediglich eine Induktionsspulenanordnung 4 untergebracht ist, die mit einer außerhalb des Reservoirkörpers 1 vorgesehenen Pumpen-/Elektronikeinheit 5, 6 verbunden ist, um diese grundsätzlich mit elektrischer Energie zu versorgen, die mit Hilfe einer geeignet vorgesehenen Batterieeinheit zwischengepuffert bzw. gespeichert werden kann. Die in dem gezeigten Ausführungsbeispiel dargestellte Adapterplatte 11 dient einer modularen Verbindung zu einer Gehäusestruktur 13, die letztlich wenigstens eine fluidische Anschlussstruktur 15 für eine Verbindungsleitung zu einer zu implantierenden Einheit aufweist, wie beispielsweise in Form eines Kompressionscuffs für eine kontrollierte Einengung eines Hohlorganes.

### Bezugszeichenliste

- 1: Reservoirkörper
- 2: Hohlkörper
- 3: Reservoirvolumen
- 4: Induktionsspulenanordnung
- 5: Pumpeneinheit
- 6: Elektronikeinheit
- 7, 7': Fluidkanal
- 8: Portkanal
- 9: Abschlusskappe
- 10: Port mit Anstichfläche
- 11: Adapterplatte
- 12: Anschlusseinheiten
- 13: Gehäuse
- 14: Batterieeinheit
- 15: Austrittsöffnung, fluidische Anschlussstruktur
- 16: Flächenport
- X: Implantierbare Einheit

## Patentansprüche

1. Implantierbarer Reservoirkörper zur bedarfsgerechten Bereitstellung eines Arbeitsmediums, das zumindest zum Inflatieren einer implantierbaren Einheit (X) dient, wobei der implantierbare Reservoirkörper (1) mit einer inflantierbaren und implantierbaren Einheit (X) verbunden ist,
**dadurch gekennzeichnet, dass** ein elastischer Hohlkörper (2) vorgesehen ist, dessen Hohlkörperwand ein Reservoirvolumen (3) umschließt,
dass eine Induktionsspulenanordnung (4) innerhalb des Reservoirvolumens (3) vorgesehen ist und/oder dass eine Induktionsspulenanordnung (4) mit der Hohlkörperwand verbunden ist,
dass die Induktionsspulenanordnung (4) mit einer inner- oder außerhalb des Hohlkörpers vorgesehenen Pumpen-/Elektronikeinheit (5, 6) verbunden ist, und dass ein aus dem Hohlkörper (2) führender Fluidkanal (7, 7') vorgesehen ist, der mit der Pumpeneinheit (5) und/oder direkt mit der zu inflatierenden implantierten Einheit (X) verbunden ist.

2. Implantierbarer Reservoirkörper nach Anspruch 1,
**dadurch gekennzeichnet, dass** der Hohlkörper (2) aus einem elastischen vorgeformten Material besteht, das im Wege eines In- und Deflatierens einer Verformung bei weitgehend konstanter Wandstärke unterliegt.

3. Implantierbarer Reservoirkörper nach Anspruch 1 oder 2,
**dadurch gekennzeichnet, dass** die Induktionsspulenanordnung (4) frei beweglich innerhalb des Hohlkörpers (2) eingebracht ist, oder
dass die Induktionsspulenanordnung (4) innerhalb der Hohlkörperwand integriert ist oder an einer Oberfläche der Hohlkörperwand fest verbunden angebracht ist.

4. Implantierbarer Reservoirkörper nach einem der Ansprüche 1 bis 3,
**dadurch gekennzeichnet, dass** im Bereich der Hohlkörperwand ein Wandabschnitt vorgesehen ist, der in Art einer Adapterplatte (11) ausgebildet ist, und
dass der Fluidkanal (7, 7') die Adapterplatte (11) durchragt oder an der Adapterplatte (11) einseitig in Form einer fluiddichten Verbindungsstruktur für den Anschluss einer zur implantierbaren Einheit (X) führenden Fluidleitung abchließt.

5. Implantierbarer Reservoirkörper nach Anspruch 4,
**dadurch gekennzeichnet, dass** an der Adapterplatte (11) eine mechanische Verbindungsstruktur für eine Adaption der implantierbaren Einheit (X) vorgesehen ist, und
dass an der Adapterplatte (11) wenigstens eine elektrische Verbindungsstruktur vorgesehen ist.

6. Implantierbarer Reservoirkörper nach einem der Ansprüche 1 bis 5,
**dadurch gekennzeichnet, dass** die Induktionsspulenanordnung (4) mit einer inner-oder außerhalb des Hohlkörpers vorgesehenen Batterieeinheit (14) verbunden ist, die zur Energieversorgung zumindest der Pumpen-/Elektronikeinheit (5, 6) dient.

7. Implantierbarer Reservoirkörper nach einem der Ansprüche 1 bis 6,
**dadurch gekennzeichnet, dass** die Pumpen-/Elektronikeinheit (5, 6) in Art einer mittels mikroelektronischer Bauelemente realisierte Baueinheit ausgebildet ist, die längs oder quer des Fluidkanals (7, 7') angebracht ist.

8. Implantierbarer Reservoirkörper nach einem der Ansprüche 1 bis 7,
**dadurch gekennzeichnet, dass** der Fluidkanal (7, 7') ein innerhalb des Reservoirvolumens (3) befindliches offenes Kanalende aufweist, das zum Schutz vor einem Verschließen mit einem innerhalb des Hohlkörpers vorhandenen Oberflächenbereiches eine Ansaugschutzkappe vorsieht.

9. Implantierbarer Reservoirkörper nach einem der Ansprüche 1 bis 8,
**dadurch gekennzeichnet, dass** in der Hohlkörperwand ein Mittel (10) vorgesehen ist, über das ein Befüllen des Hohlkörpers mit dem Arbeitsmedium erfolgt.

10. Implantierbarer Reservoirkörper nach Anspruch 9,
**dadurch gekennzeichnet, dass** das Mittel ein fluiddicht abschließbarer Port (10) ist, in Form einer planen oder einer konvex/konkaven Anstichfläche.

11. Implantierbarer Reservoirkörper nach einem der Ansprüche 1 bis 10,
**dadurch gekennzeichnet, dass** die implantierbare Einheit (X) wenigstens einen Kompressionscuff vorsieht, der durch Inflatieren mittels des seitens des Reservoirkörpers (1) bereitgestellten Arbeitsmediums zum hydraulischen Verschließen eines Hohlorganes ausgebildet ist.

12. Implantierbarer Reservoirkörper nach einem der Ansprüche 1 bis 11,
**dadurch gekennzeichnet, dass** das Arbeitsmedium eine Flüssigkeit oder ein gasförmiges Medium ist.

13. Implantierbarer Reservoirkörper nach einem der Ansprüche 1 bis 12,
**dadurch gekennzeichnet, dass** innerhalb des Reservoirvolumens (3) die Pumpeneinheit (5) und die Elektronikeinheit (6) getrennt voneinander enthalten sind.

## Claims

1. An implantable reservoir body for the need-based supply of a working medium serving at least to inflate one implantable unit (X), wherein the implantable reservoir body (1) is connected to an inflatable and implantable unit (X),
**characterized in that** an elastic hollow body (2) having a hollow body wall enclosing a reservoir volume (3) is provided,
that an induction coil arrangement (4) is provided inside the reservoir volume (3) and/or an induction coil arrangement (4) is connected to the hollow body wall,
that the induction coil arrangement (4) is connected to a pump/electronic unit (5, 6) provided inside or external of the hollow body, and that a fluid channel (7, 7') leading out of the hollow body (2) is provided which is connected to the pump unit (5) and/or directly connected to the implanted unit (X) to be inflated.

2. The implantable reservoir body according to claim 1,
**characterized in that** the hollow body (2) is made from an elastic preformed material which is subjected to deformation at a largely constant wall thickness during the course of inflation and deflation.

3. The implantable reservoir body according to claim 1 or 2,
**characterized in that** the induction coil arrangement (4) is positioned to be freely movable inside the hollow body (2) or that the induction coil arrangement (4) is integrated inside the hollow body wall or mounted in a fixed connection to a surface of the hollow body wall.

4. The implantable reservoir body according to any one of claims 1 to 3,
**characterized in that** a wall section is provided in an area of the hollow body wall which is configured as a type of adapter plate (11) and that the fluid channel (7, 7') passes through the adapter plate (11) or terminates at one side of the adapter plate (11) as a fluid-tight connecting structure for connecting a fluid line leading to the implantable unit (X).

5. The implantable reservoir body according to claim 4,
**characterized in that** a mechanical connecting structure is provided on the adapter plate (11) for adapting the implantable unit (X), and
that at least one electric connecting structure is provided on the adapter plate (11).

6. The implantable reservoir body according to any one of claims 1 to 5,
**characterized in that** the induction coil arrangement (4) is connected to a battery unit (14) provided inside or external of the hollow body which serves to supply energy to at least the pump/electronic unit (5, 6).

7. The implantable reservoir body according to any one of claims 1 to 6,
**characterized in that** the pump/electronic unit (5, 6) is configured as a type of constructional unit realized with microelectronic components which is mounted lengthwise or transversely to the fluid channel (7, 7').

8. The implantable reservoir body according to any one of claims 1 to 7,
**characterized in that** the fluid channel (7, 7') has an open channel end situated inside the reservoir volume (3) which provides an intake protection cap having a surface area inside the hollow body to protect against blocking.

9. The implantable reservoir body according to any one of claims 1 to 8,
**characterized in that** a means (10) is provided in the hollow body wall via which filling of the hollow body with a working medium occurs.

10. The implantable reservoir body according to claim 9,
**characterized in that** the means is a fluid-tight sealable port (10) in the form of a planar or a convex/concave tapping surface.

11. The implantable reservoir body according to any one of claims 1 to 10, **characterized in that** the implantable unit (X) provides for at least one compression cuff configured to hydraulically block a hollow organ by inflation with a working medium provided by the reservoir body (1).

12. The implantable reservoir body according to any one of claims 1 to 11, **characterized in that** the working medium is a liquid or a gaseous medium.

13. The implantable reservoir body according to any one of claims 1 to 12, **characterized in that** the pump unit (5) and the electronic unit (6) are incorporated inside the reservoir volume (3) separated from one another.

## Revendications

1. Corps de réservoir implantable destiné à délivrer selon les besoins un fluide de travail qui sert au moins à gonfler une unité implantable (X), le corps de réservoir implantable (1) étant relié à une unité gonflable et implantable (X), **caractérisé en ce qu'**un corps creux élastique (2) est prévu, la paroi du corps creux enfermant un volume de réservoir (3),
**en ce qu'**un dispositif à bobine d'induction (4) est prévu à l'intérieur du volume de réservoir (3) et/ou **en ce qu'**un dispositif à bobine d'induction (4) est relié à la paroi de corps creux,
**en ce que** le dispositif à bobine d'induction (4) est relié à une unité de pompage / électronique (5, 6) prévue à l'intérieur ou à l'extérieur du corps creux, et
**en ce qu'**un canal de fluide (7, 7') débouchant du corps creux (2) est prévu, lequel est relié à l'unité de pompage (5) et/ou directement à l'unité implantée à gonfler (X).

2. Corps de réservoir implantable selon la revendication 1,
**caractérisé en ce que** le corps creux (2) est composé d'un matériau élastique préformé qui subit une déformation par le biais d'un gonflage et d'un dégonflage, son épaisseur de paroi restant sensiblement constante.

3. Corps de réservoir implantable selon la revendication 1 ou 2,
**caractérisé en ce que** le dispositif à bobine d'induction (4) est logé de manière librement mobile à l'intérieur du corps creux (2), ou
**en ce que** le dispositif à bobine d'induction (4) est intégré à l'intérieur de la paroi de corps creux ou disposé sur une surface de la paroi de corps creux en y étant fermement relié.

4. Corps de réservoir implantable selon l'une des revendications 1 à 3, **caractérisé en ce qu'**une section de paroi configurée à la façon d'une plaque adaptatrice (11) est prévue dans la zone de la paroi de corps creux, et
**en ce que** le canal de fluide (7, 7') dépasse de la plaque adaptatrice (11) ou se termine au niveau de la plaque adaptatrice (11) d'un côté sous forme d'une structure de liaison étanche aux fluides pour le raccordement d'une conduite de fluide menant à l'unité implantable (X).

5. Corps de réservoir implantable selon la revendication 4,
**caractérisé en ce qu'**une structure de liaison mécanique est prévue au niveau de la plaque adaptatrice (11) pour une adaptation de l'unité implantable (X), et
**en ce qu'**au moins une structure de liaison électrique est prévue au niveau de la plaque adaptatrice (11).

6. Corps de réservoir implantable selon l'une des revendications 1 à 5, **caractérisé en ce que** le dispositif à bobine d'induction (4) est relié à une unité de batterie (14) prévue à l'intérieur ou à l'extérieur du corps creux et servant à l'alimentation en énergie d'au moins l'unité de pompage / électronique (5, 6).

7. Corps de réservoir implantable selon l'une des revendications 1 à 6, **caractérisé en ce que** l'unité de pompage / électronique (5, 6) est constituée à la façon d'une unité modulaire réalisée au moyen de modules microélectroniques et disposée le long ou en travers du canal de fluide (7, 7').

8. Corps de réservoir implantable selon l'une des revendications 1 à 7, **caractérisé en ce que** le canal de fluide (7, 7') présente une extrémité de canal ouverte située à l'intérieur du volume de réservoir (3) et munie d'un clapet anti-aspiration destiné à prévenir toute obturation avec une zone de surface présente à l'intérieur du corps creux.

9. Corps de réservoir implantable selon l'une des revendications 1 à 8, **caractérisé en ce qu'**un moyen (10) par lequel s'effectue un remplissage du corps creux avec le fluide de travail, est prévu dans la paroi de corps creux.

10. Corps de réservoir implantable selon la revendication 9,
**caractérisé en ce que** le moyen est un orifice (10) obturable de manière étanche aux fluides, sous forme d'une surface de perce plane ou convexe/concave.

11. Corps de réservoir implantable selon l'une des revendications 1 à 10, **caractérisé en ce que** l'unité implantable (X) présente au moins un ballonnet de compression qui est conçu pour obturer hydrauliquement un organe creux par gonflage au moyen du fluide de travail délivré de la part du corps de réservoir (1).

12. Corps de réservoir implantable selon l'une des revendications 1 à 11, **caractérisé en ce que** le fluide de travail est un liquide ou un fluide gazeux.

13. Corps de réservoir implantable selon l'une des revendications 1 à 12, **caractérisé en ce que** l'unité de pompage (5) et l'unité électronique (6) sont contenus séparément l'une de l'autre à l'intérieur du volume de réservoir (3).
